# EUROPÄISCHE PATENTSCHRIFT

(11) **EP 2 110 154 B1**
(45) Veröffentlichungstag und Bekanntmachung des Hinweises auf die Patenterteilung: **15.11.2017**
(21) Anmeldenummer: 09155359.4
(22) Anmeldetag: 17.03.2009
(51) Int. Cl.: A61N 1/08, A61N 1/05

(54) **Vorrichtung zur Reduktion der Störungsanfälligkeit für langgestreckte Implantate**
Device for reducing the interference susceptibility of elongate impants
Dispositif de réduction de l'occurrence de pannes pour implants allongés

(30) Priorität: 14.04.2008 DE 102008018990
(43) Veröffentlichungstag der Anmeldung: 21.10.2009
(73) Patentinhaber: Biotronik CRM Patent AG, 6341 Baar (CH)
(72) Erfinder: Weiss, Ingo, 12435, Berlin (DE); Knorr, Stefan, 10119, Berlin (DE); Maxfield, Michelle, 10967, Berlin (DE); Friedrich, Michael, 14532, Kleinmachnow (DE)
(74) Vertreter: Galander, Marcus

(56) Entgegenhaltungen:
- EP-A- 0 621 054
- WO-A-2006/055556
- WO-A-2006/097934
- US-A1- 2003 144 719
- US-A1- 2004 024 425
- US-A1- 2005 222 658
- US-B2- 6 606 513

## Beschreibung

Die Erfindung betrifft eine implantierbare Leitung mit einem langgestreckten Leitungskörper und einem sich in Längsrichtung des Leitungskörpers erstreckenden Funktionsleiter, der zur Realisierung einer medizinischen Funktion der Leitung wirkt. Derartige Leitungen sind insbesondere Stimulationselektrodenleitungen (im Folgenden auch als "Elektroden" bezeichnet) von Herzschrittmachern oder Schockelektrodenleitungen von implantierbaren Defibrillatoren, aber auch Katheter, die eine langgestreckte leitfähige Struktur enthalten.

Medizinische Implantate wie die erwähnten Schrittmacher und Defibrillatoren besitzen häufig eine elektrische Verbindung zum Körperinneren des Patienten. Eine solche Verbindung dient zur Messung von elektrischen Signalen und/oder zur Stimulation von Körperzellen. Diese Verbindung ist oft als längliche Elektrode ausgeführt. Gegenwärtig werden elektrische Signale zwischen dem Implantat und den Elektrodenkontakten wie, aber nicht beschränkt auf Spitze, Ringe, HV-Schockwendeln und Sensoren mit elektrisch gut leitenden Materialien übertragen.

Wird ein System aus Implantat und Elektrode starken Störfeldern wie EMI oder MRI ausgesetzt, so kann es zu unerwünschtem Fehlverhalten kommen, speziell einer Erwärmung von Teilen des Systems oder elektrischen Fehlfunktionen (z. B. Resets). Die Erwärmung kann zu Schädigungen von Körpergewebe oder sogar von Organen führen, wenn die erwärmten Teile direkten Kontakt zum Gewebe haben. Dies ist insbesondere bei der Elektrodenspitze der Fall.

Die Ursache für das unerwünschte Fehlverhalten ist die Wechselwirkung des Feldes mit der länglichen Leitungsstruktur der Elektrode: Die Elektrode wirkt als eine Antenne und empfängt Energie aus den umgebenden Feldern. Diese Energie auf den therapeutisch genutzten Leitungen kann die Antenne distal über die Elektrodenkontakte an das Gewebe oder proximal an das Implantat abgeben.

Die gleichen Probleme treten auch bei anderen länglichen leitfähigen Strukturen auf, deren proximales Ende nicht zwingend mit einem Implantat verbunden ist (z. B. bei Kathetern, temporäre Elektroden etc.).

Eine Elektrodenleitung, die eine Verbesserung herkömmlicher Elektrodenleitungen darstellt, ist in US 2003/0144719 A1 offenbart. Dort wird eine Elektrodenleitung gemäß des Oberbegriffes des Patentanspruchs 1 offenbart. mit einem langgestreckten Leitungskörper, einem sich in Längsrichtung des Leitungskörpers erstreckenden Funktionsleiter, der zur Realisierung einer medizinischen Funktion der Leitung wirkt, wobei zusätzlich zum Funktionsleiter ein sich mindestens über einen Abschnitt der Länge des Leitungskörpers im Wesentlichen parallel zum Funktionsleiter erstreckender Feldentkopplungsleiter vorgesehen ist, welcher eine Kopplung des Funktionsleiters mit einem äußeren Feld verringert, wobei der Feldentkopplungsleiter aus mehreren Teilstücken besteht, die verschiedene elektrische und/oder mechanische Eigenschaften besitzen und wobei die Teilstücke des Feldentkopplungsleiters eine unterteilte Struktur bilden und elektrisch miteinander verbunden sind.

Der Erfindung liegt die Aufgabe zugrunde, eine verbesserte implantierbare Leitung der genannten Art bereit zu stellen, die in starken äußeren Feldern verbesserte Eigenschaften aufweist.

Diese Aufgabe wird durch eine implantierbare Leitung mit den Merkmalen des Anspruchs 1 gelöst und zeichnet sich dadurch aus, dass die Teilstücke des Feldentkopplungsleiters über diskrete, nichtlineare und/oder Spezialbauelemente wie AMR, CMR, GMR oder TMR leitende oder dielektrische Materialien, Halbleiterstrukturen und/oder Sensoren miteinander verbunden sind.. Zweckmäßige Fortbildungen des Erfindungsgedankens sind Gegenstand der abhängigen Ansprüche.

Ein wesentlicher Gedanke der Erfindung besteht darin, den Einfluss starker äußerer Felder durch das Vorsehen eines zusätzlichen leitfähigen Elements in der implantierbaren Leitung zu reduzieren. Die zusätzlichen Leiter, so genannte Feldentkopplungsleiter, verändern die Wechselwirkung zwischen dem äußeren Feld und der Leitung so, dass sich auf der Leitung eine andere Stromverteilung ausbildet. Die ungewollten Antenneneigenschaften der Leitung verändern sich durch diese Verstimmung. Dies resultiert in einer geringeren Erwärmung der distalen Leitungskontakte. Dieser Vorteil gilt für verschiedene geometrische Formen und verschiedene Lagen der Leitung.

Diese Modifikation ist fest in der Leitung installiert oder nachrüstbar. Als Resultat verschlechtert sich die Antenneneigenschaft der Leitung und das umliegende Gewebe wird weniger erwärmt. Mit einer nachrüstbaren Modifikation kann insbesondere bei bereits implantierten Leitungen die Erwärmung der Elektrode(n) reduziert werden.

Nach Obigem ist die Erfindung besonders vorteilhaft anwendbar in einer Elektrodenleitung mit mindestens einer Elektrode und einem Elektrodenanschlusskontakt an einem Ende der Leitung, wobei der Funktionsleiter die Elektrode mit dem Elektrodenanschlusskontakt verbindet und der Feldentkopplungsleiter keine galvanische Kopplung mit dem Funktionsleiter im Bereich der oder jeder Elektrode hat.

Das proximale Ende des Feldentkopplungsleiters ist nicht notwendigerweise mit dem angeschlossenen (implantierten) Gerät bzw. dessen Funktionseinheiten, die über den Funktionsleiter die relevante medizinische Funktion realisieren, verbunden. Es kann aber durchaus eine solche Verbindung bestehen, sofern der zusätzliche Anschluss des Feldentkopplungsleiters die medizinische Funktion unterstützen kann oder jedenfalls nicht beeinträchtigt.

In einer kostengünstigen und zweckmäßigen Ausführung ist vorgesehen, dass der Feldentkopplungsleiter einen Metalldraht oder ein Metallband aufweist bzw. dass die in der Leitung vorgesehenen Mittel zum Einführen oder Anbringen eines Feldentkopplungsleiters angepasst sind, der einen Metalldraht oder ein Metallband aufweist. Bei einer alternativen Ausführung ist vorgesehen, dass der Feldentkopplungsleiter eine langgestreckte leitfähige Polymerstruktur oder eine leitfähige Flüssigkeit in einem Leiterkanal (Innenlumen des Leitungskörpers) aufweist.

Sofern der Feldentkopplungsleiter aus Metall oder einem Polymeren gebildet ist, ist unter mechanischen Gründen ggf eine mehradrige Ausbildung und speziell eine mehradrige Seilstruktur vorteilhaft. Des Weiteren kann dann der Feldentkopplungsleiter eine wendelförmige, mäanderförmig, gefaltete oder selbstähnliche Feinstruktur aufweisen, welche optional an eine korrespondierende Feinstruktur des Funktionsleiters eingefügt ist oder um eine definierte Struktur des Funktionsleiters herum ausgebildet ist.

In einer weiteren Ausführung ist der Feldentkopplungsleiter und/oder der Funktionsleiter als metallische Abscheidung auf einem isolierenden Substrat ausgeführt, wobei insbesondere der Feldentkopplungsleiter und der Funktionsleiter isoliert voneinander auf ein und demselben Substrat ausgebildet sind. Speziell kann hierbei das Substrat als biegsamer Schlauch ausgebildet sein. Auch ein solches flexibles Substrat, das unter dem Aspekt einer leichten Verlegung im Körper vorteilhaft ist, lässt sich mit modernen Abscheidungstechniken leicht mit einer hinreichend dicken Leiterschicht versehen.

Unter verschiedenen Anwendungsaspekten und im Hinblick auf speziell elektrische und mechanische Anforderungen der erfindungsgemäß verbesserten Leitung ist eine Vielzahl weiterer Ausgestaltungen bzw. Abwandlungen des Konzeptes möglich.

So kann der Feldentkopplungsleiter aus mehreren Teilstücken bestehen, die verschiedene elektrische und/oder mechanische Eigenschaften besitzen, und/oder eine Isolierung des Feldentkopplungsleiters kann inhomogen sein oder ihre Eigenschaften können sich über die Längserstreckung ändern. Eines oder beide Enden eines aus mehreren Leitern bestehenden Feldentkopplungsleitersystems können an einem Ende oder beiden Enden elektrisch miteinander verbunden sein, oder es können Teilstücke einer unterteilten Struktur elektrisch miteinander verbunden sein, und zwar ggf. über diskrete Bauelemente (Widerstände, Kondensatoren, Induktivitäten), durch leitende oder dielektrische Materialien (Kunststoffe, Metalle, Keramik), Halbleiterstrukturen, nichtlineare Bauelemente oder auch Sensoren (wie etwa Thermoelemente, Thermistoren, Feldstärkesensoren für elektrische oder magnetische Felder etc.). Auch eine Verbindung über Spezial-Bauelemente, die einen Riesenmagnetwiderstand (GMR), einen anisotropen Magnetwiderstand (AMR), einen kolossalen Magnetwiderstandseffekt (CMR) oder einen magnetischen Tunnelwiderstand (TMR) haben, ist in Spezial-Ausführungen möglich.

Bei einem Aufbau aus mehreren Teilstücken können diese ggf. einen elektrischen Schwingkreis bilden, dessen Parameter in vorbestimmter Weise auf die Feldparameter eines im Gebrauchszustand einwirkenden externen Feldes abgestimmt sein können.

Das proximale Ende kann bei leitfähiger Ausführung Kontakt zu mindestens einem der Kontakte eines proximalen Elektrodensteckers (Tip- oder Ringkontakt) oder auch zu einer Metallfläche haben, die Teil eines Implantats ist, ggf. aber von dessen übrigem Gehäuse isoliert sein kann. Die Verbindung am proximalen Ende kann auch ein oder mehrere passive oder aktive elektronische Bauelemente beinhalten. Das proximale Ende des Feldentkopplungsleiters kann auch dann leitfähig ausgeführt sein, wenn dieser nicht mit einem zugehörigen medizinischen Implantat verbunden wird - andererseits ist aber für gewisse Anwendungen eine proximal isolierte Ausführung zu bevorzugen.

Um der vorgeschlagenen Leitung geeignete mechanische Eigenschaften zu verleihen, kann etwa vorgesehen sein, dass der distale Bereich des Feldentkopplungsleiters durch geeignete Materialwahl, Geometrie und ggf. Isolierung in besonders hohem Maße flexibel ist, um eine unerwünschte Versteifung des distalen Bereiches der Leitung insgesamt zu vermeiden. In diesem Sinne vorteilhaft ist auch die weiter oben bereits erwähnte Ausführung mit einem flüssigen Leiter (oder ausschließlich aus flüssigen Leitern), insbesondere in einem hochgradig flexiblen Schlauch. Ähnlich vorteilhafte Eigenschaften hat auch ein oberflächlich dünn metallisierter hochflexibler Schlauch.

Unter mechanischen und implantationstechnischen Aspekten vorteilhaft ist des Weiteren eine Ausführung des Feldentkopplungsleiters, bei dem dessen Oberfläche einen geringen Reibungsbeiwert hat, also etwa mit einer PTFE-Beschichtung o. ä. versehen ist.

In einer speziellen Ausgestaltung kann das proximale Ende des Feldentkopplungsleiters einen federelastischen Abschnitt oder eine entsprechende Komponente (etwa Lamellen, Wendeln, etc.) aufweisen, um auch unter mechanischer Wechselbelastung zuverlässig elektrischen Kontakt zu einem angrenzenden Leiter - etwa in einer Norm-Anschlussbuchse mit Zusatzkontakt - herzustellen.

Zur schnellen Unterscheidung erfindungsgemäßer Leitungen von herkömmlichen Leitungen kann ein geeigneter Röntgen- oder Ultraschall-Marker oder eine andere Markierung vorgesehen sein, die in einem bildgebenden Verfahren deutlich sichtbar ist.

In einer aus derzeitiger Sicht bevorzugten Ausführung ist der Feldentkopplungsleiter innerhalb des Leitungskörpers angeordnet oder anordenbar. Grundsätzlich kann aber der Feldentkopplungsleiter außen am Leitungskörper angebracht oder anbringbar sein. Es versteht sich, dass speziell bei sehr langen Leitungen zur Erleichterung der Verlegung eine Unterbringung des Feldentkopplungsleiters im Leitungskörper bevorzugt ist. Speziell zur nachträglichen Verbesserung der Störfeld-Performance von bereits vorhandenen Leitungen, die nicht allzu lang sind oder die in einem Unterimplantations-Gesichtspunkten unkritischen Bereich liegen, kann aber auch eine nachträgliche Anbringung des Feldentkopplungsleiters von außen möglich und vorteilhaft sein.

In einer weiteren Ausführung der Erfindung sind im Inneren des Leitungskörpers Isoliermittel zur Isolierung des Feldentkopplungsleiters vom Funktionsleiter bzw. der oder jeder Elektrode vorgesehen, oder ein zum nachträglichen Einführen oder Anbringen bereitgestellter Feldentkopplungsleiter weist eine integrale Isolierung auf.

Für eine fest in der Leitung installierte Feldentkopplungs-Struktur sind weitere Ausführungen möglich:
- eine Leitungsstruktur, die zusammen mit einer therapeutisch und/oder messtechnisch genutzten Leitung zusammen als eine Wendel gewickelt ist (z. B. in der Helix des Innenleiters);
- eine Leitungsstruktur, die parallel zu und in möglichst nahem physischen (aber nicht galvanischen) Kontakt mit einer therapeutisch und/oder messtechnisch genutzten Leitung geführt oder gewickelt ist (z. B. um die Helix des Innenleiters, um die Helix des Außenleiters oder um ein Seil herum);
- eine Leitungsstruktur, die teilweise um den Draht der Helix einer therapeutisch und/oder messtechnisch genutzten Leitung gewickelt ist;
- eine Leitungsstruktur, deren Aufbau mindestens teilweise selbstähnlich ist, so dass eine lange elektrische Leitung in einem kurzen Aufbau untergebracht werden kann;
- eine Leitungsstruktur, die sich innerhalb der Elektrode (exzentrisch oder zentriert) oder außerhalb der Elektrode (koaxial, exzentrisch) befindet;
- für die als zweite grundsätzliche Ausführung der Erfindung vorgesehene "nachrüstbare" Feldentkopplungs-Struktur ist eine Ausführung möglich;
- die auf die benötigte Länge (abhängig von der Länge der betreffenden Elektrode) gekürzt werden kann;
- die auf eine Einführhilfe gesteckt werden kann und so einfacher ins Lumen einer Elektrode geschoben werden kann, die also z. B. als Schlauch ausgeführt werden kann, in dessen Innenlumen ein Mandrin geschoben werden kann,
- die proximal und/oder distal gekürzt werden kann;
- die proximal eine spezielle Form besitzt, um mit einem passenden Werkzeug aus dem Innenlumen der Elektrode wieder entfernt zu werden (z. B. zum Repositionieren der Elektrode mit einem Mandrin),
- die teilweise aus einem Material, z. B. Metall oder Kunststoff, besteht, dessen Nachgiebigkeit mit steigender Temperatur steigt (so ist die einzuführende Struktur anfangs steif und wird beim Erreichen der Körpertemperatur weicher - die Versteifung der Elektrode ist gering);
- die aus mehreren Leitern besteht, die an mehreren Stellen miteinander verbunden sind (Diese Verbindung sorgt für eine Versteifung gegenüber der gleichen Leiteranordnung ohne Verbindung. Die Verbindung kann nachträglich gelöst werden z. B. durch das Auflösen der verbindenen Substanz. So kann die Steifigkeit der Leitungsstruktur nach dem Einführen in das Innenlumen einer Elektrode verringert werden, indem z. B. die Salzkristalle, die die Drähte eines Seiles verbinden durch die Injektion von Wasser aufgelöst werden);
- die fest im Innenlumen der Elektrode fixiert werden kann, um eine Verschiebung und eine mögliche Perforation des Gewebes zu vermeiden (insbesondere bei Coronary Sinus Elektroden mit durchgehender, distaler Lumenöffnung);
- die aus einem Gel besteht;
- die aus einem zweikomponentigen, aushärtenden Kunststoff besteht;
- die aus einer leitfähigen Flüssigkeit, die direkt ins Innenlumen der Elektrode injiziert wird. In diesem Fall besteht sowohl zur Elektrodenspitze als auch zur Innenwendel ein elektrischer Kontakt;
- wobei die Injektion des flüssigen Leiters mit einem langen Schlauch erfolgt, der anfangs ins distale Ende des Innenlumens geschoben wird.

Eine Alternative zur distal isolierten Leitungsstruktur ist eine distal unisolierte Leitungsstruktur, die in eine Elektrode geschoben wird, deren distales Ende innen isoliert ist. Diese Isolierung kann durch ein dielektrisches Material gewährleistet werden, das fest in der Elektrode eingebaut ist oder nachträglich montiert wird (z. B. bei der Implantation).

Das erfindungsgemäße Vorsehen einer zusätzlichen Feldentkopplungs-Struktur in einer medizinischen Leitung ermöglicht den Bau von MR-sicheren Implantaten, die selbst bei den starken elektromagnetischen Feldern (z. B. während einer MR Untersuchung) eine nur moderate Erwärmung der Elektrodenspitze aufweisen. Es ermöglicht auch die nachträgliche Modifikation bereits implantierter Elektroden, um ein MR-sicheres System zu erhalten, insbesondere von Leitungen, die nicht mit einem aktiven Implantat verbunden sind und ungenutzt im Körper liegen.

Vorteile und Zweckmäßigkeiten der Erfindung ergeben sich im Übrigen aus der nachfolgenden Beschreibung von speziellen Ausführungen anhand der Figuren. Von diesen zeigen:
- Fig. 1A bis 1C: eine Gesamtansicht aus zwei Detailansichten (distaler und proximaler Abschnitt) einer Stimulationselektrodenleitung gemäß einer Ausführungsform der Erfindung,
- Fig. 2: eine Detailansicht des distalen Abschnitts einer modifizierten Ausführung der Elektrodenleitung,
- Fig. 3: eine Detailansicht des proximalen Endes einer weiteren Ausführung der erfindungsgemäßen Elektrodenleitung,
- Fig. 4A bis 4C: Querschnittsdarstellungen verschiedener Ausführungen des Feldentkopplungsleiters einer erfindungsgemäßen Stimulationselektrodenleitung,
- Fig. 5A und 5F: schematische Querschnittsdarstellungen erfindungsgemäßer bipolarer Stimulationselektrodenleitungen, bei denen der Feldentkopplungsleiter verschiedene Ausführungen bzw. Positionen hat,
- Fig. 6 bis 8: schematische Darstellungen weiterer Ausführungen bzw. Anordnungen des Feldentkopplungsleiters,
- Fig. 9A und 9B: eine perspektivische Ausschnittsdarstellung sowie eine schematische perspektivische Detaildarstellung einer weiteren Ausführung des Feldentkopplungsleiters,
- Fig. 10: eine schematische Längsschnittdarstellung des proximalen Abschnitts einer weiteren erfindungsgemäßen Stimulationselektrodenleitung,
- Fig. 11A bis 11C: schematische Darstellungen verschiedener Ausgestaltungen des Stimulationsgeräteanschlusses erfindungsgemäßer Stimulationselektrodenleitungen,
- Fig. 12: eine schematische Längsschnittdarstellung einer weiteren Ausführungsform der erfindungsgemäßen Stimulationselektrodenleitung, in einer Phase ihrer Herstellung, und
- Fig. 13A und 13B: schematische Darstellungen einer weiteren konstruktiven Ausführung des Feldentkopplungsleiters.

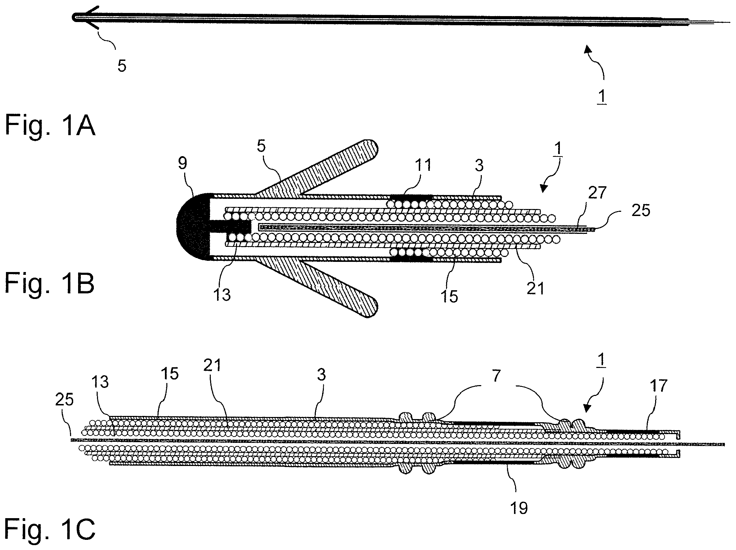

Die Figuren 1A bis 1C zeigen eine erfindungsgemäße Stimulationselektrodenanordnung 1 in schematischen Längsschnittdarstellungen, und zwar eine Gesamtansicht (Fig. 1A) und Detailansichten des distalen (Fig. 1B) und proximalen Teiles (Fig. 1C). Die Elektrodenleitung umfasst einen Leitungskörper 3, an den nahe dem distalen Ende 2 Finnen 5 zur Verankerung als Stimulationselektrodenleitung im Trabekelwerk eines Herzens und nahe dem proximalen Ende Dichtlippen bzw. -ringe 7 zur flüssigkeitsdichten Abdichtung in einer IS1-Anschlussbuchse eines Stimulationsgerätes (Herzschrittmachers) angeformt sind. In den Leitungskörper 3 eingearbeitet sind als distaler Abschluss eine im Wesentlichen halbkugelförmige Tip-Elektrode 9 und proximal hiervon beabstandet eine Ring-Elektrode 11.

Die Elektroden 9, 11 sind über eine innere und eine äußere, koaxial angeordnete und gewendelte Elektrodenzuleitung 13 bzw. 15 angeschlossen, die am proximalen Ende der Leitung mit einem ersten und zweiten Steckeranschlusskontakt 17 bzw. 19 verbunden sind. Die Elektrodenzuleitungen 13, 15 sind durch einen Isolierschlauch 21 gegeneinander isoliert. Sie dienen als Funktionsleiter der Elektrodenleitung 1. In deren Längsachse verläuft ein zusätzlicher, gestreckter Leiter 25 mit einem Isoliermantel 27, der die Funktion des weiter oben bereits erläuterten Feldentkopplungsleiters hat.

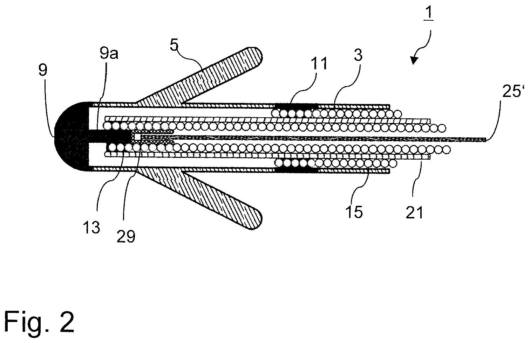

Die in Fig. 2 als Detailansicht gezeigte modifizierte Elektrodenleitung 1' hat im Wesentlichen den gleichen Aufbau wie die Elektrodenleitung 1 nach Fig. 1A bis 1C, der insoweit auch mit den gleichen Bezugsziffern bezeichnet ist. Sie unterscheidet sich von jener durch unisolierten zentralen Feldentkopplungsleiter 25' und das zusätzliche Vorsehen einer Isolierhülse 29 innerhalb des distalen Endabschnittes der inneren Elektrodenzuleitung 13, in Kontakt mit einem stiftartigen, nach innen gerichteten Fortsatz 9a der Spitzen-Elektrode 9. Diese Hülse ermöglicht grundsätzlich den Einsatz eines unisolierten Drahtes als Feldentkopplungsleiter, ohne dass eine Verbindung mit geringer Impedanz zwischen jenem und der Tip-Elektrode besteht.

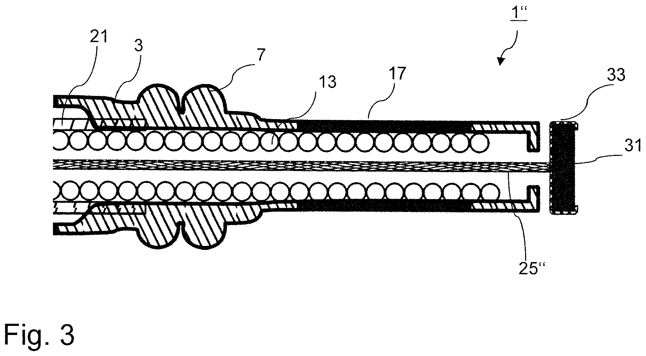

In einer weiter modifizierten Elektrodenleitung 1', die in Fig. 3 dargestellt ist und in den übrigen Teilen der Elektrodenleitung 1 aus Fig. 1A bis 1C entspricht, hat der zentrale Feldentkopplungsleiter 25" am proximalen Ende eine in der Draufsicht kreisförmige Anschlussplatte 31, deren Umfang und eine Stirnseite abgesehen von der Kontaktfläche mit dem Feldentkopplungsleiter 25" mit einer isolierenden Beschichtung 33 bedeckt sind.

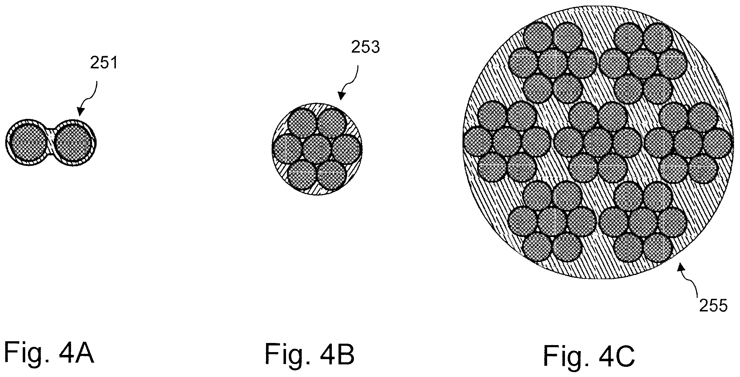

In Fig. 4A bis 4C sind als verschiedene Ausführungen eines Feldentkopplungsleiters eine zweiadrige Leitung 251, ein siebenadriges Seil 253 und ein geflochtenes 7 x 7-Seil 255, jeweils mit (nicht gesondert bezeichneter) Isolierung gezeigt. Die Seile können in sich inhomogen aufgebaut sein und ggf. einen Kern aus einem anderen Material als die übrigen Stränge besitzen.

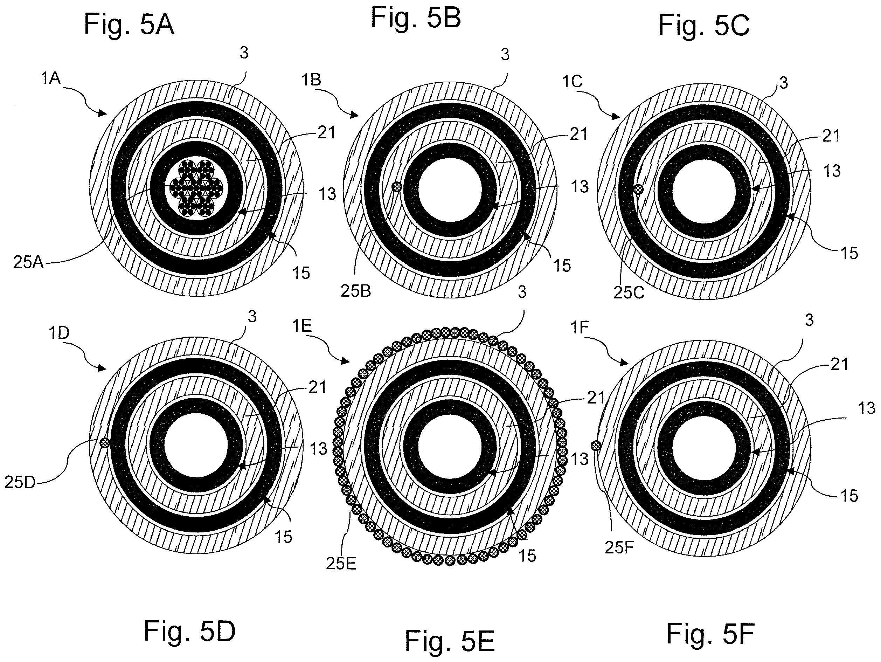

Die Fig. 5A bis 5F zeigen verschiedene Aufbauten von erfindungsgemäßen Stimulationselektrodenleitungen 1A bis 1F, deren herkömmliche Komponenten - Leitungskörper 3, Innen-Zuleitung 13, Außen-Zuleitung 15 und Isolierschlauch 21 - übereinstimmend vorgesehen sind. Bei der Leitung 1A ist in einem zentralen Lumen (nicht gesondert bezeichnet) eine mehradrige Seilstruktur 25A als Feldentkopplungsleiter vorgesehen und bei den Leitungen 1B (Fig. 5B), 1C (Fig. 5C), 1D (Fig. 5D) und 1F (Fig. 5F) jeweils ein einzelner Draht 25B oder 25C oder 25D oder 25F exzentrisch in verschiedenen Positionen bezüglich Innen- und Außen-Zuleitung bzw. außen am Leitungskörper 3. Die Leitung 1E nach Fig. 5E hat einen den gesamten Umfang des Leitungskörpers 3 umgebenden, vieladrigen Feldentkopplungs-Mantelleiter 25E.

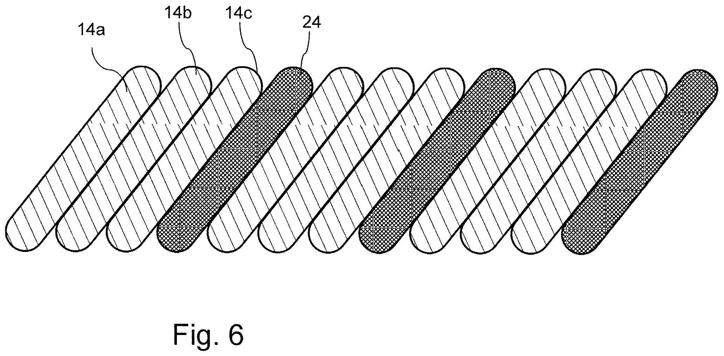

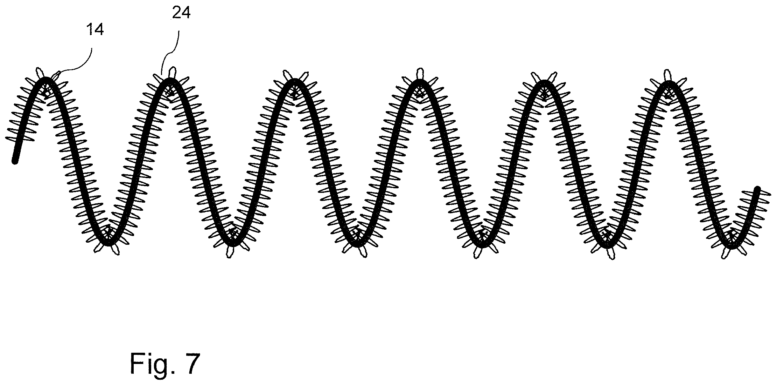

Der Feldentkopplungsleiter kann insgesamt oder partiell eine wendelförmige, mäanderförmige, gefaltete oder selbstähnliche Struktur besitzen und beispielsweise auch gemeinsam mit Funktionsleitern in eine mehradrige Wendel gewickelt sein, wie Fig. 6 zeigt. Hier ist eine vieradrige Wendel aus drei Funktionsleitern 14a bis 14c und einem Feldentkopplungsleiter 24 gewickelt. In der in Fig. 7 gezeigten weiteren Ausführung ist ein Feldentkopplungsleiter 24 als Helix um eine ihrerseits gewendelte therapeutisch genutzte Leitung (Funktionsleiter 14) gewickelt.

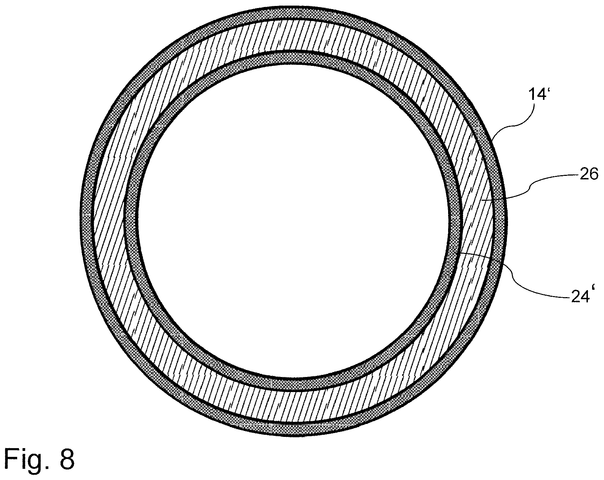

Als weitere Ausführung ist in Fig. 8 eine aus dünnen Metallschichten beidseits eines Substrates bestehende Leitungsstruktur gezeigt, und zwar in Gestalt eines Isolierschlauches 26 mit einer als Funktionsleiter dienenden Außenbeschichtung 14' und einer als Feldentkopplungsleiter dienenden Innenbeschichtung 24'. In modifizierten Ausführungen kann die Funktionszuordnung der Leitschichten auch umgekehrt sein, oder der Schlauch kann insgesamt lediglich einen zweischichtigen Feldentkopplungsleiter darstellen.

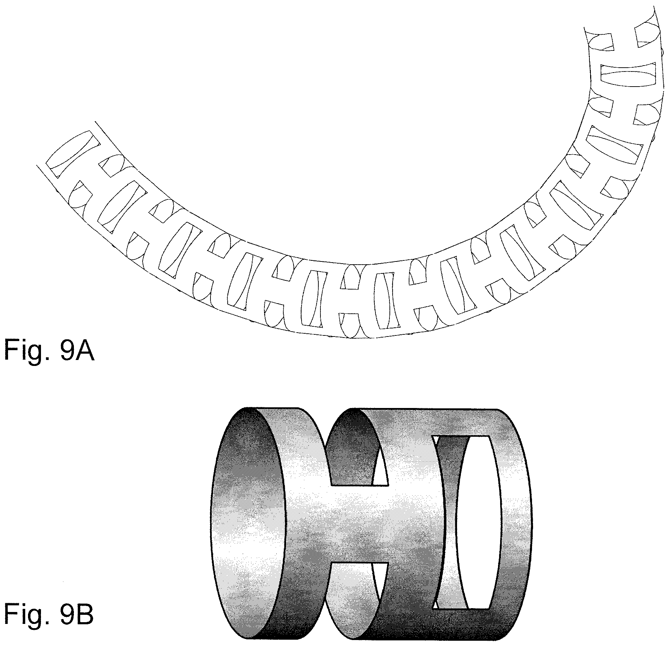

Die Metallisierung kann eine Feinstruktur besitzen, die z. B. durch ein lithographisches Verfahren erzeugt wird. Der Träger der Metallisierung kann eine Helix sein oder eine andere Feinstruktur besitzen. Der Träger sollte durch seine Form sehr elastisch sein, um die Steifigkeit der Elektrode nicht zu stark zu erhöhen. Die Steifigkeit eines Schlauches kann z. B. durch mehrere Aussparungen variiert werden, beispielsweise durch Einschnitte, die das Flächenträgkeitsmoment der Struktur stark reduzieren, ohne die Längssteifigkeit zu stark zu beeinflussen. Fig. 9A und 9B zeigen eine Ausführungsform einer derartigen Schlauchstruktur mit gegeneinander versetzten, periodisch wiederkehrenden Ausschnitten.

Eine Schlauchstruktur kann auch bei geringer Eigensteifigkeit gut mit einem Führungsdraht ins Innenlumen der Elektrode eingeführt werden. Am distalen Ende kann eine Struktur vorgesehen werden, die zusammen mit einer passenden weiteren Struktur im Innern der Elektrode einen Haltemechanismus ausbildet. Beim Einführen des Schlauches mit einem Führungsdraht wird der Schlauch in der Spitze gehalten und der Führungsdraht kann entfernt werden, ohne die Position des Schlauches zu verändern. Der Haltemechanismus sollte lösbar sein, um den Feldentkopplungsleiter wieder entfernen zu können.

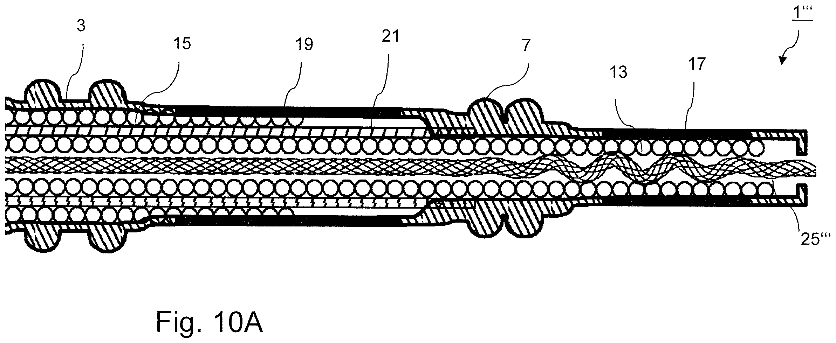

Fig. 10 zeigt, als weitere Abwandlung der in Fig. 1A bis 1C gezeigten Ausführung, eine Elektrodenleitung 1"', in deren Innerem ein Feldentkopplungsleiter 25"' mit gewendeltem proximalen Abschnitt verläuft, welcher einen gewollten elektrischen Kontakt mit der Innen-Zuleitung 15 herstellt.

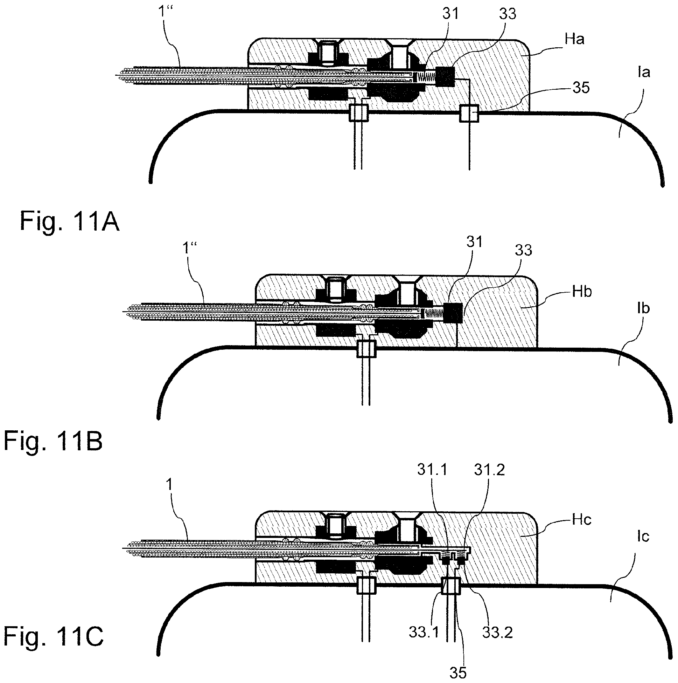

In Fig. 11A bis 11C sind verschiedene Varianten des Anschlusses einer erfindungsgemäßen Elektrodenleitung 1" gemäß Fig. 3 bzw. einer Leitung 1 gemäß Fig. 1 an ein medizinisches Implantat schematisch dargestellt. Die gezeigten Implantate Ia, Ib bzw. Ic unterscheiden sich in der Ausbildung ihres jeweiligen Headers Ha, Hb bzw. Hc. Die Anschlussbuchsen bauen jeweils auf dem IS1-Standard auf und sind kompatibel zu diesem, bieten aber die zusätzliche Möglichkeit, einen Feldentkopplungsleiter der Elektrodenleitung zu kontaktieren.

Fig. 11A zeigt ein Implantat Ia mit einer Header Ha, der den Feldentkopplungsleiter über eine Druckfeder 31 mit einem dafür vorgesehenen Kontakt 33 elektrisch verbindet, wobei eine Gehäusedurchführung 35 den Kontakt in das Gehäuseinnere des Implantats weiterführt. Fig. 11B zeigt eine ähnliche Ausführung, bei der allerdings die Gehäusedurchführung entfällt und der Feldentkopplungsleiter über den Kontakt 33 direkt mit dem Implantatgehäuse verbunden ist. Wie Fig. 11C zeigt, ist auch eine separate Kontaktierung mehrerer Einzelleiter einer Feldentkopplungsleiterstruktur möglich, und zwar über separate Kontaktfedern 31.1, 31.2 und Kontakte 33.1, 33.2 im Header Hc.

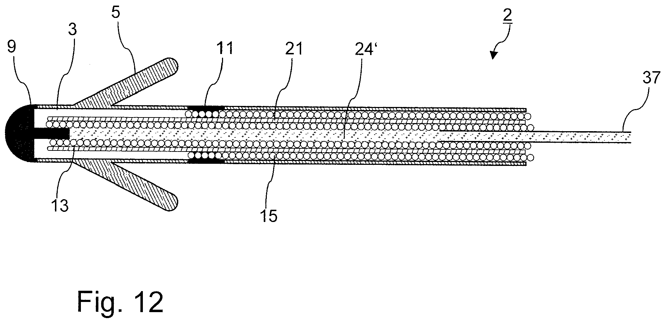

Eine spezielle Ausführungsform des Feldentkopplungsleiters, wie in Fig. 12 gezeigt, ist eine leitfähige Flüssigkeit, die ins Innenlumen der Elektrode gespritzt werden kann. Diese Flüssigkeit kann z. B. eine wässrige Lösung von Salzen sein. Um eine gute Längsleitfähigkeit der Flüssigkeitssäule zu erreichen, sollte sie ohne Unterbrechungen (z. B. Luftblasen) vom distalen bis zum proximalen Ende reichen. Dies kann man z. B. mittels einer Injektion durch einen langen Schlauch hindurch erreichen: der Schlauch wird in die Elektrodenspitze geführt und anschließend während des Injizierens aus dem Innenlumen gezogen. Fig. 12 zeigt eine entsprechende Elektrodenleitung 2, deren Struktur weitgehend mit der Elektrodenleitung 1 aus Fig. 1A bis 1C übereinstimmt und insoweit auch mit den gleichen Bezugsziffern bezeichnet ist. Der Unterschied besteht darin, dass statt eines metallischen zentralen Feldentkopplungsleiters im Innenlumen eine leitfähige Flüssigkeit 24' vorgesehen ist, die in die bereits verlegte Elektrodenleitung nachträglich über einen Schlauch 37 eingebracht wird.

Um die Leitfähigkeit der Lösung zeitlich konstant zu halten, sollte die Diffusion der Ionen aus der Elektrode heraus sehr langsam erfolgen. Dies kann durch geeignete Sperrschichten oder genügend große Ionen gewährleistet werden.

Um das Einführen des Feldentkopplungsleiters zu erleichtern, ist eine steife Struktur wünschenswert. Der platzierte Feldentkopplungsleiter sollte, jedoch eine möglichst geringe Steifigkeit besitzen. Beide Ziele können erreicht werden, wenn die Steifigkeit veränderlich ist. Dies kann z. B. durch Materialien mit einem so genannten Gedächtniseffekt erreicht werden.

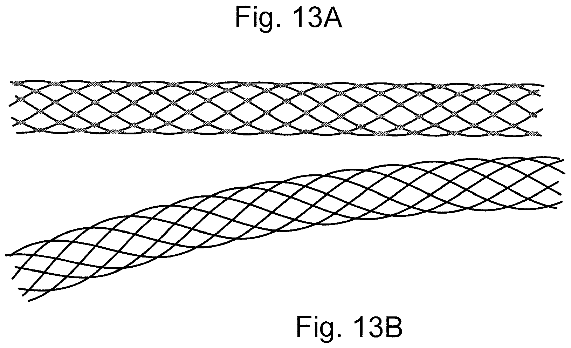

Alternativ können mehrere Teilstrukturen des Feldentkopplungsleiters mit einer löslichen Substanz versteift werden, die nach der Implantation aufgelöst werden. So wird die Steifigkeit reduziert. Als Beispiel ist in Fig. 13A und 13B ein geflochtenes Seil dargestellt, dessen Einzelfasern aus verschiedenen Materialien bestehen können. Die einzelnen Fasern sind z. B. mit Zucker- oder Salzkristallen verbunden, die nach der Implantation mit Wasser gelöst werden können. Fig. 13A zeigt das steife Geflecht vor der Implantation mit den Versteifungen und Fig. 13B das nachgiebige Geflecht mit den gelösten Versteifungen.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Leitung (1) mit einem langgestreckten Leitungskörper, einem sich in Längsrichtung des Leitungskörpers erstreckenden Funktionsleiter (13, 15), der zur Realisierung einer medizinischen Funktion der Leitung wirkt, wobei zusätzlich zum Funktionsleiter (13, 15) ein sich mindestens über einen Abschnitt der Länge des Leitungskörpers im Wesentlichen parallel zum Funktionsleiter erstreckender Feldentkopplungsleiter (25) vorgesehen ist, welcher eine Kopplung des Funktionsleiters (13, 15) mit einem äußeren Feld verringert, wobei der Feldentkopplungsleiter aus mehreren Teilstücken besteht, die verschiedene elektrische und/oder mechanische Eigenschaften besitzen und wobei die Teilstücke des Feldentkopplungsleiters eine unterteilte Struktur bilden und elektrisch miteinander verbunden sind,
**dadurch gekennzeichnet, dass**
die Teilstücke des Feldentkopplungsleiters über diskrete, nichtlineare und/oder Spezialbauelemente wie AMR, CMR, GMR oder TMR, leitende oder dielektrische Materialien, Halbleiterstrukturen und/oder Sensoren miteinander verbunden sind.

2. Implantierbare Leitung nach Anspruch 1, ausgebildet als Elektrodenleitung mit mindestens einer Elektrode und einem Elektrodenanschlusskontakt an einem Ende der Leitung, wobei der Funktionsleiter (13, 15) die Elektrode mit dem Elektrodenanschlusskontakt verbindet und der Feldentkopplungsleiter (25) keine galvanische Kopplung mit dem Funktionsleiter (13, 15) im Bereich der oder jeder Elektrode hat.

3. Implantierbare Leitung nach einem oder mehreren der vorhergehenden Ansprüche, wobei der Feldentkopplungsleiter (25) einen separaten elektrischen Anschluss am proximalen Ende der Leitung aufweist.

4. Implantierbare Leitung nach Anspruch 1, wobei die Teilstücke des Feldentkopplungsleiters einen elektrischen Schwingkreis bilden, dessen Parameter in vorbestimmter Weise auf die Feldparameter eines im Gebrauchszustand einwirkenden externen Feldes abgestimmt sind.

5. Implantierbare Leitung nach einem oder mehreren der vorhergehenden Ansprüche, wobei der Feldentkopplungsleiter (25) eine mehradrige Seilstruktur oder einen oder mehrere Metalldrähte oder ein oder mehrere Metallbänder aufweist.

6. Implantierbare Leitung nach einem der vorangehenden Ansprüche, wobei der Feldentkopplungsleiter (25) eine wendelförmige, mäanderförmig, gefaltete oder selbstähnliche Feinstruktur aufweist, welche optional an eine korrespondierende Feinstruktur des Funktionsleiters (13, 15) eingefügt ist.

7. Implantierbare Leitung nach einem oder mehreren der vorhergehenden Ansprüche, wobei der Feldentkopplungsleiter (25) aus mehreren Leitern besteht, bei dem ein oder beide Enden der Leiter an einem oder beiden Enden des Feldentkopplungsleiters elektrisch miteinander verbunden sind.

8. Implantierbare Leitung nach einem der Ansprüche 1 bis 4, wobei der Feldentkopplungsleiter (25) und/oder der Funktionsleiter (13, 15) als metallische Abscheidung auf einem isolierenden Substrat ausgeführt ist, wobei insbesondere der Feldentkopplungsleiter (25) und der Funktionsleiter (13, 15) isoliert voneinander auf ein und demselben Substrat ausgebildet sind.

9. Implantierbare Leitung nach einem der Ansprüche 1 bis 3, wobei der Feldentkopplungsleiter (25) eine langgestreckte leitfähige Polymerstruktur oder eine leitfähige Flüssigkeit in einem Leiterkanal aufweist.

10. Implantierbare Leitung nach einem oder mehreren der vorhergehenden Ansprüche, wobei im Inneren des Leitungskörpers Isoliermittel (21) zur Isolierung des Feldentkopplungsleiters (25) vom Funktionsleiter (13, 15) bzw. von der oder jeder Elektrode vorgesehen sind oder ein zum nachträglichen Einführen oder Anbringen ausgebildeter Feldentkopplungsleiter (25) eine integrale Isolierung aufweist, wobei die Isolation des Feldentkopplungsleiters vorzugsweise zusätzlich inhomogen ist, um ihre Eigenschaften in Längserstreckung veränderbar zu gestalten.

11. Implantierbare Leitung nach einem der vorangehenden Ansprüche, wobei der Feldentkopplungsleiter (25) innerhalb des Leitungskörpers anordenbar ist.

12. Implantierbare Leitung (1) nach Anspruch 1, wobei Mittel zum Einführen oder Anbringen des Feldentkopplungsleiters (25) derart vorgesehen sind, dass der Feldentkopplungsleiter (25) sich mindestens über einen Abschnitt der Länge des Leitungskörpers im Wesentlichen parallel zum Funktionsleiter (13, 15) erstreckt.

## Claims

1. An implantable lead (1) with a long, stretched out lead body, a functional lead (13, 15) that extends in the longitudinal direction of the lead body and that acts to realize a medical function of the lead, a field decoupling conductor (25) being provided in addition to the functional lead (13, 15), the field decoupling conductor (25) extending essentially parallel to the functional lead over at least one section of the length of the lead body and reducing a coupling of the functional lead (13, 15) with an external field and consisting of multiple parts that possess different electrical and/or mechanical properties and that form a subdivided structure and are electrically connected with one another,
**characterized in that**
the parts of the field decoupling conductor are connected with one another through discrete, nonlinear and/or special components such as AMR, CMR, GMR or TMR, conductive or dielectric materials, semiconductor structures, and/or sensors.

2. An implantable lead according to claim 1, made in the form of an electrode lead with at least one electrode and an electrode connection contact at one end of the lead, the functional lead (13, 15) connecting the electrode with the electrode connection contact and the field decoupling conductor (25) having no galvanic coupling with the functional lead (13, 15) in the area of the electrode or every electrode.

3. An implantable lead according to one or more of the preceding claims, wherein the field decoupling conductor (25) has a separate electrical connection at the proximal end of the lead.

4. An implantable lead according to claim 1, wherein the parts of the field decoupling conductor form an oscillating electrical circuit whose parameters are matched in a predetermined manner to the field parameters of an external field that has an effect in the operational state.

5. An implantable lead according to one or more of the preceding claims, wherein the field decoupling conductor (25) has a multiconductor cable structure or one or more metal wires or one or more metal strips.

6. An implantable lead according to one or more of the preceding claims, wherein the field decoupling conductor (25) has a helical, meandering, folded, or self-similar fine structure, which is optionally inserted into a corresponding fine structure of the functional lead (13, 15).

7. An implantable lead according to one or more of the preceding claims, wherein the field decoupling conductor (25) consists of multiple conductors, one or both ends of the conductors being electrically connected with one another at one or both ends of the field decoupling conductor.

8. An implantable lead according to any one of claims 1 through 4, wherein the field decoupling conductor (25) and/or the functional lead (13, 15) is/are made by metallic deposition on an insulating substrate, in particular wherein the field decoupling conductor (25) and the functional lead (13, 15) are insulated from one another on one and the same substrate.

9. An implantable lead according to any one of claims 1 through 3, wherein the field decoupling conductor (25) has a long, stretched-out conductive polymer structure or a conductive liquid in a conductor channel.

10. An implantable lead according to one or more of the preceding claims, wherein insulation means (21) are provided inside the lead body to insulate the field decoupling conductor (25) from the functional lead (13, 15) or from the electrode or from every electrode, or a field decoupling conductor (25) designed for subsequent introduction or attachment has an integrated insulation, the insulation of the field decoupling conductor preferably additionally being non-homogeneous, to make its properties changeable along its longitudinal extension.

11. An implantable lead according to any one of the preceding claims, wherein the field decoupling conductor (25) can be arranged within the lead body.

12. An implantable lead (1) according to claim 1, wherein means are provided to introduce or attach the field decoupling conductor (25) so that the field decoupling conductor (25) extends essentially parallel to functional lead (13, 15) over at least a section of the length of the lead body.

## Revendications

1. Ligne implantable (1) avec un corps de ligne s'étendant en longueur, un conducteur fonctionnel (13, 15) s'étendant dans le sens longitudinal du corps de ligne, qui agit pour la réalisation d'une fonction médicale de la ligne, où, outre la ligne fonctionnelle (16, 15), est prévu au moins un conducteur de découplage de champ (25) s'étendant sur une partie de la longueur du corps de ligne de manière essentiellement parallèle par rapport au conducteur fonctionnel, lequel diminue un couplage du conducteur fonctionnel (13, 15) avec un champ extérieur, où le conducteur de découplage de champ est constitué de plusieurs pièces qui possèdent des caractéristiques électriques et/ou mécaniques diverses et où les pièces du conducteur de découplage de champ forment une structure divisée et sont reliées électriquement entre elles,
**caractérisée en ce que**
les pièces du conducteur de découplage de champ sont reliées entre elles par le biais de composants discrets, non linéaires et/ou spéciaux comme des matériaux AMR, CMR, GMR ou TMR, conducteurs ou diélectriques, des structures de semiconducteurs et/ou des capteurs.

2. Ligne implantable selon la revendication 1, conçue en tant que ligne d'électrode avec au moins une électrode et une borne de contact d'électrode à une extrémité de la ligne, où le conducteur fonctionnel (13, 15) relie l'électrode avec la borne de contact d'électrode et le conducteur de découplage de champ (25) n'a pas de couplage galvanique avec le conducteur fonctionnel (13, 15) dans la zone de l'électrode ou de chaque électrode.

3. Ligne implantable selon l'une ou plusieurs des revendications précédentes, où le conducteur de découplage de champ (25) présente une connexion électrique séparée à l'extrémité proximale de la ligne.

4. Ligne implantable selon la revendication 1, dans laquelle les pièces du conducteur de découplage de champ forment un circuit oscillant électrique dont les paramètres sont ajustés d'une manière prédéterminée aux paramètres de champ d'un champ externe agissant dans l'état d'utilisation.

5. Ligne implantable selon l'une ou plusieurs des revendications précédentes, dans laquelle le conducteur de découplage de champ (25) présente une structure en corde à plusieurs fils ou un ou plusieurs fils métalliques ou une ou plusieurs bandes métalliques.

6. Ligne implantable selon l'une ou plusieurs des revendications précédentes, dans laquelle le conducteur de découplage de champ (25) présente une structure fine en forme d'ondulation, en forme de méandres, pliée ou analogue, laquelle est éventuellement incorporée à une structure fine du conducteur fonctionnel (13, 15).

7. Ligne implantable selon l'une ou plusieurs des revendications précédentes, dans laquelle le conducteur de découplage de champ (25) est constitué de plusieurs conducteurs, chez lequel une ou les deux extrémités du conducteur de découplage de champ sont reliées ensemble électriquement.

8. Ligne implantable selon l'une des revendications 1 à 4, dans laquelle le conducteur de découplage de champ (25) et/ou le conducteur fonctionnel (13, 15) est conçu sous forme d'un dépôt métallique sur un substrat isolé, où notamment, le conducteur de découplage de champ (25) et le conducteur fonctionnel (13, 15) sont isolés l'un par rapport à l'autre et sont constitués du même substrat.

9. Ligne implantable selon l'une des revendications 1 à 3, dans laquelle le conducteur de découplage de champ (25) présente une structure de polymère conducteur allongée ou présente un liquide conducteur dans un canal de conducteur.

10. Ligne implantable selon l'une ou plusieurs des revendications précédentes, dans laquelle à l'intérieur du corps de ligne, des moyens d'isolation (21) sont prévus pour l'isolement du conducteur de découplage de champ (25) du conducteur fonctionnel (13, 15), respectivement, de l'électrode ou de chaque électrode ou qu'un conducteur de découplage de champ (25) conçu pour une insertion ou une mise en place ultérieure présente une isolation intégrale, où l'isolation du conducteur de découplage de champ est en outre de préférence non homogène pour concevoir ses caractéristiques dans l'extension longitudinale modifiables.

11. Ligne implantable selon l'une des revendications précédentes, dans laquelle le conducteur de découplage de champ (25) peut être disposé à l'intérieur du corps de ligne.

12. Ligne implantable (1) selon la revendication 1, dans laquelle des moyens pour l'insertion ou la mise en place du conducteur de découplage de champ (25) sont prévus de telle manière que le conducteur de découplage de champ (25) s'étend au moins sur une partie de la longueur du corps de ligne de manière essentiellement parallèle par rapport au conducteur fonctionnel (13, 15).
